# EUROPEAN PATENT APPLICATION

(11) **EP 3 964 257 A1**
(43) Date of publication of application: **09.03.2022**
(21) Application number: 20194500.3
(22) Date of filing: 04.09.2020
(51) Int. Cl.: A61N 1/04, A61B 5/257, A61B 5/259

(54) **STIMULATING / SENSING ELECTRODE COMPRISING A PRINTABLE ADHESIVE**

(71) Applicant: Henkel AG & Co. KGaA, 40589 Düsseldorf (DE)
(72) Inventor: Negele, Carla, 41472 Neuss (DE); Roschek, Tobias, 42699 Solingen (DE); Goethel, Frank, 09125 Chemnitz (DE); van der Meulen, Inge, 5521 WM Eersel (NL)

(57) **Abstract**

The present invention relates to a stimulation or a sensing electrode comprising an ionically and/or electrically conductive pressure sensitive adhesive composition comprising a) an ionic liquid and/or carbon black; and b) a resin selected from the group consisting of a (meth)acrylate resin, a polyurethane resin, a silicone resin and mixtures thereof. The electrodes according to the present invention are used in skin applications transferring an electrical signal to the body via the skin or in skin applications as a contact medium as part of electrodes measuring bio-signals from the skin.

## Description

### Technical field of the invention

The present invention relates to a stimulating or sensing electrode comprising an ionically and/or electrically conductive pressure sensitive adhesive allowing prolongated stimulation or sensing times without skin irritation and loss of signal quality.

### Background of the invention

Various kinds of electrodes are used to measure bio signals such as electrocardiography (ECG), electroencephalography (EEG) and electromyography (EMG). Equally various kinds of electrodes are used to provide an electrical stimulation. By the term electrical stimulation is meant herein any application where an electrical signal is transferred to the body via the skin. Examples of such applications are for example transcutaneous electrical nerve stimulation (TENS), electrical muscle stimulation (EMS), functional electrical stimulation (FES), wound care, electrosurgery, defibrillation, electrodermal activity (EDA) and total body electrical conductivity.

For example, currently used ECG electrodes are connected to the skin via a gel, which acts as an electrolyte and transfers the body signal to the electrode. However, they dry out overtime and cannot be used for prolongated measurements. Most of the cases, they are not recommended for use longer than 24 hours. In addition, they do not have long storage times, most of the cases one month at the maximum after opening, and furthermore, they need a special packaging preventing them from drying out.

Especially currently used gel electrodes have high salt concentrations, which are needed for low impedances and good signal quality, however, at the same time they cause skin irritation at many patients. Furthermore, these electrodes contain relatively high quantity of water. The high-water content is one reason why these electrodes tend to dry out, and therefore, cannot be used for long-term measurements (maximum of three days). Because the signal quality decreases along decreasing water content.

There are also tab electrodes currently on the market, which are attached to the skin via a gel-type adhesive. These electrodes do not need an additional skin adhesive, since the gel itself is adhering to the skin. However, these electrodes also comprise a salt and water, and can dry out overtime and therefore, are not suitable for prolongated measurements. The cohesion of the adhesive is often poor in these electrodes, leading to cohesive failure upon removal of the electrode.

In another electrode solution, the electrode comprises adhesives comprising the combination of carbon black and a salt. An electrophoretic alignment of conductive fillers is required in order to obtain sufficient impedances in this solution. However, this electrophoretic activation step makes the electrode production expensive and complicated.

Electrical stimulation can feature significantly high currents compared to measuring bio-signals. However, high local current densities can feel unpleasant or even cause a pain and damage, therefore, higher electrode areas should be used, especially for stimulation of deeper lying muscles where higher currents for stimulation are required. Larger electrode areas are obviously more critical for current distribution because the resistance of the conductive layer will be more critical. Furthermore, high local current densities may be caused by dissimilarities in the skin resistance (e.g. hair follicles, sweat glands).

Many of the currently used stimulation gel electrodes can be used more than once to save material and costs, however the gel in the electrode gets dirty after several use, and this is not acceptable in a hospital environment and physiotherapy practices.

In the production process of electrodes comprising a pressure sensitive adhesive, first the conductive layer has to be printed on a substrate which is usually done via screen printing. As most pressure sensitive adhesives are solvent based, usually the second step would be the coating of the adhesive between release liners on a conventional coater including a drying oven. The third step is to laminate the pressure-sensitive adhesive onto the conductive layer. All three steps require different equipment and are usually performed at different companies, and transportation and logistics between the manufacturing locations are needed to produce the electrodes.

Therefore, there is a need for electrodes for use in stimulation and sensing applications, which can be used for a prolongated time without loss of signal or adhesion or getting dirty, while providing good current distribution and not drying out or sensitizing or irritating the skin. Further, there is a need for a screen printable adhesive for the stimulation or sensing electrodes that would allow the printing of the adhesive onto the conductive layer directly after the printing of the conductive layer.

### Summary of the invention

The present invention relates to a stimulation or a sensing electrode comprising an ionically and/or electrically conductive pressure sensitive adhesive composition comprising a) an ionic liquid and/or carbon black; and b) a resin selected from the group consisting of a (meth)acrylate resin, a polyurethane resin, a silicone resin and mixtures thereof.

The present invention also relates to use of a stimulation or a sensing electrode according to the present invention in skin applications transferring an electrical signal to the body via the skin or in skin applications as a contact medium as part of electrodes measuring bio-signals from the skin.

### Detailed description of the invention

In the following passages the present invention is described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an" and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

As used herein, the term *"consisting of"* excludes any element, ingredient, member or method step not specified.

The recitation of numerical end points includes all numbers and fractions subsumed within the respective ranges, as well as the recited end points.

All percentages, parts, proportions and then like mentioned herein are based on weight unless otherwise indicated.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

All references cited in the present specification are hereby incorporated by reference in their entirety.

Unless otherwise defined, all terms used in disclosing the invention, including technical and scientific terms, have the meaning as commonly understood by one of the ordinary skilled in the art to which this invention belongs to. By means of further guidance, term definitions are included to better appreciate the teaching of the present invention.

A dry stimulation or sensing electrode according to the present invention comprises an ionically and/or electrically conductive pressure sensitive adhesive (PSA) with low impedance, giid current distribution and good skin compatibility.

The present invention pertains to a stimulation electrode, which does not require a gel or hydrogel, therefore the term "dry electrode" is employed for the stimulation electrode according to the present invention as well.

By the term dry stimulation electrode and dry sensing electrode is meant herein an electrode which does not contain a substantial amount of solvent and is free of water. The dry stimulating or sensing electrode according to the present invention comprises less than 1% of weight of the total weight of the adhesive a solvent, preferably less than 0.1% and more preferably less than 0.01%.

By the term "screen printable" is meant herein that the an ionically and/or electrically conductive adhesive used in the present invention is screen printable, which is secured by using resins which can be screen printed or be dissolved into a screen printable solvent. The screen printable solvent has a high boiling point, low vapor pressure and a high enough flashpoint.

A dry sensing or stimulation electrode according to the present invention offers a solution to a long-term use in stimulating or sensing, wherein long-term use is possible with stable impedance and adhesion. In contrast to gel-type electrodes currently in the market it cannot dry out and it does not lead to skin irritation. The dry electrode according to the present invention has longer shelf-life and a storage of the electrodes is possible even after opening of the package. Further, the dry electrode according to the present invention provides comfort due to the very thin and dry layer leading to electrodes one does not feel in contrast to state of the art gel electrodes that are relatively heavy and swell the skin. In combination with thin, flexible, and breathable substrates like polyurethane films the electrodes according to the present invention will allow a comfortable continuous long-term application. Further, the electrode according to the present invention provides better current distribution. In addition, because the adhesive used in the present invention is a screen printable adhesive this allows the printing of the adhesive onto the conductive layer directly after the printing of the conductive layer. This will lead to the use of the same equipment for all necessary production steps making the complete process simpler, more cost and time effective and more sustainable.

The present invention refers to a stimulation or a sensing electrode comprising an ionically and/or electrically conductive pressure sensitive adhesive composition comprising a) an ionic liquid and/or carbon black; and a resin selected from the group consisting of a (meth)acrylate resin, a polyurethane resin, a silicone resin and mixtures thereof; b) a conductive layer, which is in contact with said ionically and/or electrically conductive pressure sensitive adhesive layer; c) optionally a substrate, which is in contact with the conductive layer; and d) optionally a release liner, which is in contact with the conductive pressure sensitive adhesive layer.

The pressure sensitive adhesive used in a dry stimulating or sensing electrode according to the present invention is an ionically and/or electrically conductive pressure sensitive adhesive. The ionically and/or electrically conductive pressure sensitive adhesive used in the present invention is a screen printable adhesive.

An ionically and/or electrically conductive pressure sensitive adhesive composition according to the present invention may comprise an ionic liquid, preferably a non-toxic, non-irritating ionic liquid leading to ionic conductivity.

In one embodiment the pressure sensitive adhesive according to the present invention comprises an ionic liquid, and therefore, the adhesive is ionically conductive.

In another embodiment the pressure sensitive adhesive according to the present invention comprises an ionic liquid and carbon black, and therefore, the adhesive is ionically and electrically conductive.

More specifically, an ionically and/or electrically conductive pressure sensitive adhesive composition according to the present invention comprises an ionic liquid selected from the group consisting of imidazolium acetates, imidazolium sulfonates, imidazolium chlorides, imidazolium sulphates, imidazolium phosphates, imidazolium thiocyanates, imidazolium dicyanamides, imidazolium benzoates, imidazolium triflates, choline triflates, choline saccharinate, choline sulfamates, pyridinium acetates, pyridinium sulfonates, pyridinium chlorides, pyridinium sulphates, pyridinium phosphates, pyridinium thiocyanates, pyridinium dicyanamides, pyridinium benzoates, pyridinium triflates, pyrrolidinium acetates, pyrrolidinium sulfonates, pyrrolidinium chlorides, pyrrolidinium sulphates, pyrrolidinium phosphates, pyrrolidinium thiocyanates, pyrrolidinium dicyanamides, pyrrolidinium benzoates, pyrrolidinium triflates, phosphonium acetates, phosphonium sulfonates, phosphonium chlorides, phosphonium sulphates, phosphonium phosphates, phosphonium thiocyanates, phosphonium dicyanamides, phosphonium benzoates, phosphonium triflates, sulfonium acetates, sulfonium sulfonates, sulfonium chlorides, sulfonium sulphates, sulfonium phosphates, sulfonium thiocyanates, sulfonium dicyanamides, sulfonium benzoates, sulfonium triflates, ammonium acetates, ammonium sulfonates, ammonium chlorides, ammonium sulphates, ammonium phosphates, ammonium thiocyanates, ammonium dicyanamides, ammonium benzoates, ammonium triflates and mixtures thereof.

Preferably the ionic liquid is selected from the group consisting of 1-ethyl-3-methylimidazolium acetate, 1-ethyl-3-methylimidazolium methanesulfonate, 1-ethyl-3-methylimidazolium trifluoromethanesulfonate, 1-ethyl-3-methylimidazolium chloride, 1-ethyl-3-methylimidazolium ethyl sulphate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluoromethanesulfonate, choline saccharinate, choline acesulfamate, choline *N-*cyclohexylsulfamate, tris(2-hydroxyethyl)methylammonium methyl sulphate, 1-ethyl-3-methylimidazolium tetrafluoroborate, 1-allyl-3-ethylimidazolium bis(trifluoromethylsulfonyl)imide, choline acetate, choline trifluoromethanesulfonate and mixtures thereof.

More preferably the ionic liquid is selected from the group consisting of 1-ethyl-3-methylimidazolium benzoate, 1-ethyl-3-methylimidazolium tetrafluoroborate, 1-ethyl-3-methylimidazolium methanesulfonate, 1-ethyl-3-methylimidazolium chloride, 1-ethyl-3-methylimidazolium trifluoromethanesulfonate, choline trifluoromethanesulfonate, 1-ethyl-3-methylimidazolium acetate, choline acetate, 1-ethyl-3-methylimidazolium diethylphosphate, 1-allyl-3-methylimidazolium bis(trifluoromethylsulfonyl)imide, 1-ethyl-3-methylimidazolium ethyl sulphate, 1-ethyl-3-methylimidazolium thiocyanate, 1-ethyl-3-methylimidazolium dicyanamide, choline saccharinate, choline acesulfamate, and mixture thereof.

Above mentioned ionic liquids are preferred because they have good solubility to the selected resins and low toxicity.

Suitable commercially available ionic liquids for use in the present invention include, but are not limited to Basionics ST80, Basionics Kat1, Basionics BC01, Basionics VS11, Basionics VS03, and Efka IO 6785, all from BASF.

An ionically and/or electrically conductive pressure sensitive adhesive used in a stimulation or a sensing electrode according to the present invention may comprise an ionic liquid from 0.1 to 35% by weight of the total weight of the composition, preferably from 2 to 25%, and more preferably from 5 to 15%.

If the quantity of the ionic liquid is too low, the adhesive may not show any ionic conductivity and the signal may be lost, whereas too high quantity may not provide improvement in signal quality but may increase the chances of skin irritation and decrease the adhesion properties.

An ionically and/or electrically conductive pressure sensitive adhesive composition according to the present invention may further comprise an ionic conductivity promoter, preferably a non-toxic, non-irritating ionic conductivity promoter leading to additional ionic conductivity. The ionic conductivity promoter is preferably semi-solid or solid under room temperature and can be dissolved into the ionic liquid. It has good compatibility with the resin according to the present invention.

Preferably said ionic conductivity promoter is selected from the group consisting of choline chloride, choline bitartrate, choline dihydrogen citrate, choline phosphate, choline gluconate, choline fumarate, choline carbonate, choline pyrophosphate and mixture thereof.

Suitable commercially available ionic conductivity promoters for use in the present invention include, but are not limited to Choline Chloride from TCI Chemicals.

An ionically and/or electrically conductive pressure sensitive adhesive used in a stimulation or a sensing electrode according to the present invention may comprise an ionic conductivity promoter from 0.1 to 35% by weight of the total weight of the composition, preferably from 0.5 to 25%, and more preferably from 1 to 15%.

If the quantity of the ionic conductivity promoter is too low, the pressure sensitive adhesive may not show any ionic conductivity and the signal may be lost, whereas too high quantity may not provide improvement in signal quality but may increase the chances of skin irritation and decrease the adhesion properties.

An ionically and/or electrically conductive pressure sensitive adhesive composition according to the present invention may comprise carbon black. Carbon black is preferred due to its lower cost and is does not cause skin irritation while providing adequate conductivity.

In one embodiment the pressure sensitive adhesive according to the present invention comprises carbon black, and therefore, the adhesive is electrically conductive.

In another embodiment the pressure sensitive adhesive according to the present invention comprises carbon black and an ionic liquid, and therefore, the adhesive is electrically and ionically conductive.

Various carbon blacks are suitable for use in the present invention, but it is preferred that the carbon black has an oil absorption number from 70 ml/100g to 600 ml/100g, more preferably from 100 ml/100g to 500 ml/100g and more preferably from 150 ml/100g to 450 ml/100g, wherein said oil absorption number is measured according to ASTM D2414.

The carbon black may be further characterised by its iodine number. Preferably the carbon black has an iodine number from 30 mg/g to 1700 mg/g, more preferably from 100 mg/g to 1500 mg/g and even more preferably from 200 mg/g to 1400 mg/g, wherein said iodine number is measured according to ASTM D1510.

Suitable commercially available carbon blacks for use in the present invention include, but are not limited to Ensaco 250G, Timrex KS6 from Timcal, Printex XE2B and Printex L6 from Necarbo, C-Nergy Super C65 from Imerys and Vulcan XC72R and Vulcan XCmax22 from Cabot.

An ionically and/or electrically conductive pressure sensitive adhesive used in a stimulation or a sensing electrode according to the present invention may comprise carbon black from 0.01 to 30% by weight of the total weight of the composition, preferably from 1 to 25%, and more preferably from 5 to 20%.

If the quantity of the carbon black is too low, it may lead to poor conductivity, whereas too high quantity may lead to loss of adhesion properties.

An ionically and/or electrically conductive pressure sensitive adhesive composition according to the present invention comprises a resin selected from the group consisting of a (meth)acrylate resin, polyurethane resin, a silicone resin, and mixtures thereof.

An ionically and/or electrically conductive adhesive used in the present invention is screen printable adhesive. This is secured by using resins which can be either screen printed or can be dissolved into a screen printable solvent.

In one embodiment said resin is a (meth)acrylate resin. More specifically a (meth)acrylate resin formed from the monomers selected from the group consisting of methyl (meth)acrylate, ethyl (meth)acrylate, n-propyl (meth)acrylate, iso-propyl (meth)acrylate, n-butyl (meth)acrylate, iso-butyl (meth)acrylate, amyl (meth)acrylate, hexyl (meth)acrylate, ethylhexyl (meth)acrylate, octyl (meth)acrylate, isooctyl (meth)acrylate, decyl (meth)acrylate, dodecyl (meth)acrylate, hydroxyethyl (meth)acrylate, hydroxypropyl (meth)acrylate, hydroxybutyl (meth)acrylate, (meth)acrylic acid, benzyl (meth)acrylate, cyclohexyl (meth)acrylate, glycidyl (meth)acrylate, (meth)acrylamide, vinyl acetate, N-vinyl pyrrolidone, N-vinyl caprolactame, N-tertiary octyl acrylamide, dimethyl acrylamide, diacetone acrylamide, N-tertiary butyl acrylamide, N-isopropyl acrylamide, N-vinyl acetamide, N-vinyl formamide, acrylonitrile, vinyl ether and mixtures thereof, preferably selected from the group consisting of methyl (meth)acrylate, butyl (meth)acrylate, ethylhexyl acrylate, hydroxyethyl (meth)acrylate, hydroxypropyl (meth)acrylate, vinyl acetate, (meth)acrylic acid mixtures thereof.

Above monomers are preferred not only because of their commercial availability but because they provide balanced adhesion and cohesion while providing breathable adhesive. Further, they are soluble into a screen printable solvent.

Suitable commercially available (meth)acrylate resins for use in the present invention include, but are not limited to Loctite Duro-TAK 222A, Loctite Duro-TAK 87-202A; Loctite Duro-TAK 87-402A; Loctite Duro-TAK 73-626A LOCTITE Duro-TAK UV 26105, LOCTITE Duro-TAK UV 4040, LOCITE Duro-TAK 4606 and Loctite Duro-TAK 235A from Henkel.

In another embodiment said resin is a polyurethane resin. More specifically a polyurethane resin formed from an isocyanate and polyol prepolymer.

Preferably the isocyanate is selected from the group consisting of toluene-2,4-diisocyanate, methylene diphenyl diisocyanate, polymeric methylene diphenyl diisocyanate, hexamethylene diisocyanate, isophorone diisocyanate, 4,4'-diisocyanatodicyclohexylmethane and mixtures thereof.

Preferably the polyol is selected from polyester polyol, polyether polyol, renewable polyol sources such as castor oil and mixtures thereof, more preferably the polyol is selected from the group consisting of polypropylene glycol, polyethylene glycol, polytetramethylene glycol, castor oil and mixtures thereof.

In a highly preferred embodiment said polyurethane resin is formed from an aliphatic NCO-terminated prepolymer based on hexamethylene diisocyanate and from polyether polyol based on a tri- or tetra-functional starter molecule.

In one embodiment thermoplastic polyurethane resins are used as resin in the ionically and/or electrically conductive pressure sensitive adhesive according to the present invention.

Suitable commercially available polyurethane resins for use in the present invention include but are not limited to Baymedix^{®} AP501 and Baymedix^{®} AR602 from Covestro.

Yet in another embodiment said resin is a silicone resin or a silicone acrylate hybrid resin.

An ionically and/or electrically conductive pressure sensitive adhesive used in a stimulation or a sensing electrode according to the present invention may comprise said resin from 30 to 98% by weight of the total weight of the composition, preferably from 50 to 95% and more preferably from 75 to 92%.

Lower resin quantity may lead to poor adhesion properties and is not beneficial to film forming properties, whereas too high quantity may lead to a poor conductivity.

An ionically and/or electrically conductive pressure sensitive adhesive composition according to the present invention may further comprise a polyether polyol. Preferably, the polyether polyol is selected from polyethylene glycol (PEG), polypropylene glycol (PPG), polytetramethylene glycol (PTMG) and mixtures thereof. The applicant has found out that addition of polyether polyol is an exceptionally good host for ionic conductivity due to the open and flexible molecule chains, and therefore, has a positive impact on the impedance. The applicant has found out that already a small quantity of polyether polyol has a positive impact, which is beneficial regarding the skin compatibility of the composition.

Preferably, the polyether polyol may have a weight averaged molecular weight (M_{w}) from 300 to 1000 g/mol, preferably from 350 to 750 g/mol and more preferably from 380 to 420 g/mol, wherein the molecular weight is measured by gel permeation chromatography according to DIN 55672-1:2007-08 with THF as the eluent.

Suitable commercially available polyether polyols for use in the present invention include but are not limited to Kollisolv PEG 400 from BASF.

An ionically and/or electrically conductive pressure sensitive adhesive used in a stimulation or a sensing electrode according to the present invention may comprise polyether polyol from 0.01 to 50% by weight of the total weight of the composition, preferably from 0.1 to 25% and more preferably from 1 to 20%.

Too high polyether polyol quantity may lead to loss of adhesion properties.

An ionically and/or electrically conductive pressure sensitive adhesive composition according to the present invention may further comprise a solvent. Suitable solvent for use in the present invention is a screen printable solvent, meaning that said solvent has a high boiling point and low vapor pressure and a flashpoint high enough (preferably, the flash point of the solvent is from 70 to 120 °C) while dissolving the resin. If the boiling point of the solvent is too low and vapor pressure is too high and flash point is too low, the solvent evaporates quickly, and the adhesive dries too fast on the screen during the printing process. In other words, the solvent should not dry on the screen, but it should not take too long to dry a coating either.

Suitable solvent for use in the present invention can be selected from the group consisting of alcohols, ketones, esters, glycol esters, glycol ethers, ethers, acetates, carbonates, and mixtures thereof.

Preferably, said solvent is selected from the group consisting of dipropylene glycol, ethylene glycol, diethylene glycol, triethylene glycol, hexylene glycol, 1-methoxy-2-propanol, diacetone alcohol, 2-ethyl-1,3-hexanediol, tridecanol, 1,2-octanediol, butyldiglycol, alpha-terpineol or beta-terpineol, 2-(2-butoxyethoxy)ethyl acetate, 2,2,4-trimetyl-1,3-pentanediol diisobutyrate, 1,2-propylene carbonate, carbitol acetate, butyl carbitol acetate, butyl carbitol, ethyl carbitol acetate, 2-phenoxy ethanol, hexylene glycol, dibutylphthalate, dibasic ester (DBE), dibasic ester 9 (DBE-9), dibasic ester 7 (DBE-7), 2-(2-butoxyethoxy)ethanol, dipropylene glycol, 2-(2-ethoxyethoxy)ethyl acetate, (2-butoxyethyl)acetate, ethyl levulinate, butyl levulinate, cyrene, γ-valerolactone and mixtures thereof.

Suitable commercially available solvents for use in the present invention include but are not limited to Butyl CARBITOL^{™} and Butyl CARBITOL^{™} Acetate from DOW Inc..

An ionically and/or electrically conductive pressure sensitive adhesive used in a stimulation or a sensing electrode according to the present invention may comprise a solvent from 10 to 90% by weight of the total weight of the composition, preferably from 20 to 80%, and more preferably from 30 to 70%.

If the quantity of the solvent is too low, this may lead to problems in processability since the viscosity is too high, and the resin may not be fully soluble. Whereas too high quantity may lead to loss of functionality, and the viscosity of the adhesive is too low to process.

For the sake of the clarity, it is noted that the pressure sensitive adhesive according to the present invention may comprise a solvent. A pressure sensitive adhesive film formed from said adhesive is used in the dry stimulating or sensing electrode according to the present invention and does not comprise substantial amount of solvent. As described above the dry stimulating or sensing electrode according to the present invention comprises less than 1% of weight of the total weight of the adhesive a solvent, preferably less than 0.1% and more preferably 0.01%.

When the electrode is a stimulating electrode, the ionically and/or electrically conductive pressure sensitive adhesive composition according to the present invention ideally has an impedance in a similar range than the skin impedance. Whereas when the electrode is directed to sensing, the ionically and/or electrically conductive pressure sensitive adhesive composition according to the present invention ideally has an impedance lower than the skin impedance. The impedance depends on the area of the electrode and underlying skin.

An ionically and/or electrically conductive pressure sensitive adhesive composition according to the present invention has impedance value below 1,000,000 Ohm at 10 Hz, preferably below 100,000 Ohm at 10 Hz, and more preferably below 40,000 Ohm at 10 Hz, wherein said impedance is measured by connecting two electrodes coated each with 25 µm of an ionic conductive pressure sensitive adhesive having a contact area of 0.25 cm². However, the impedance value is not below 5000 Ohm at 10 Hz for adhesives comprising ionic liquid and not below 1000 Ohm at 10 Hz for adhesives comprising carbon black.

An ionically and/or electrically conductive pressure sensitive adhesive composition according to the present invention has high breathability. Good breathability is obtained if the water can penetrate easily through the adhesive layer. To achieve this effect, a quite polar resin is required, in this occasion, the OH-functionalities support and improve the breathability.

Adhesive according to the present invention has preferably a breathability value of about 4600 g/m² in 24 hours. As a comparison a standard acrylic PSA has a breathability value of about 2000 g/m² in 24 hours. The breathability is measured through a moisture vapor transmission rate (MVTR) measurement according to ASTM D1653.

The present invention relates to a dry stimulation or sensing electrode formed from the ionically and/or electrically conductive pressure sensitive adhesive composition as described above. The dry film formation can be done by coating the ionically and/or electrically conductive pressure sensitive adhesive composition on a supporting substrate (such as a film). And subsequently drying the film in an oven at for example 120°C for 15 minutes to remove the solvent and form a dry film of the ionically and/or electrically conductive pressure sensitive adhesive on the supporting substrate.

The known method used for preparing pressure-sensitive adhesives can be used. Specifically, examples include roll coating, gravure coating, reverse coating, roll brushing, spray coating, and air knife coating methods, immersing and curtain coating method, screen printing, stencil printing and extruding coating method with a die coater.

A stimulation or a sensing electrode according to the present invention further comprises a conductive layer, which is in contact with said ionically and/or electrically conductive pressure sensitive adhesive layer; optionally a substrate, which is in contact with the conductive layer; optionally a release liner, which is in contact with the conductive pressure sensitive adhesive layer; and optionally a contact between said electrode and stimulation device.

The electrode according to the present invention contains a conductive layer, preferably only one conductive layer.

In one embodiment the conductive layer is selected from copper, silver, gold, aluminium, Ag/AgCI, or a carbon layer or mixtures thereof.

In one embodiment the conductive layer has a thickness of 0.1 to 500 µm, or 0.5 to 150 µm, or 1 to 25 µm, or 1 to 20 µm.

In another embodiment, the conductive layer is a metal, preferably with a thickness of 10 to 500 µm, or 25 to 150 µm. Preferably, the metal is a copper, silver, gold, or aluminium layer.

In one embodiment, the conductive layer of the dry sensing electrode is Ag/AgCl layer when the pressure sensitive adhesive according to the present invention comprises an ionic liquid. Whereas in another embodiment the pressure sensitive adhesive according to the present invention comprises carbon black, and the conductive layer of the dry sensing electrode is carbon layer.

In one embodiment, the conductive layer of the stimulating electrode is Ag/AgCl layer, and the pressure sensitive adhesive according to the present invention comprises an ionic liquid and/or carbon black.

In a further embodiment the conductive layer is the only conductive layer contained in the electrode in addition to the conductive pressure sensitive adhesive.

In preferred embodiments, the electrode according to the present invention contains a substrate. In one embodiment the substrate is a flexible film, preferably selected from polyolefin films, polycarbonate films, thermoplastic polyurethane (TPU) films, silicone films, woven films, non-woven films, or paper films, in particular polyethylene films, polypropylene films, polyethylene terephthalate films or thermoplastic polyurethane films or foams such as polyurethane foams, polyolefin foams and polycarbonate foams.

In another embodiment the substrate has a thickness of 10 to 500 µm, or 25 to 150 µm.

In order to package the electrode and avoid that the adhesive layer sticks to the package, the electrode may contain a release liner on the surface of the adhesive layer which is later applied to the area which should be measured. All known release liners in the art are suitable, in one embodiment the release liner is selected from siliconized paper release liner or plastic release liner.

As already stated above the dry sensing or stimulating electrode according to the present invention does not require a gel/hydrogel. Therefore, in one embodiment, the electrode is essentially free from a hydrogel, preferably does not contain more than 0.5 wt.-%, or 0.1 wt.-%, or 0.01 wt.-% of a hydrogel, or does not contain a hydrogel, based on the total weight of the electrode.

In another embodiment the dry sensing or stimulating electrode according to the present invention is essentially free from an aqueous electrolyte paste, preferably does not contain more than 0.5 wt.-%, or 0.1 wt.-%, or 0.01 wt.-% of an aqueous electrolyte paste, or does not contain an aqueous electrolyte paste, based on the total weight of the electrode.

It is desired that sensing electrodes according to the present invention have a low impedance, whereas it is desired that the stimulating electrodes have a good current distribution.

A stimulation or a sensing electrode according to the present invention is used in skin applications transferring an electrical signal to the body via the skin or in skin applications as a contact medium as part of electrodes measuring bio-signals from the skin.

More specifically, the stimulation electrode according to the present invention is used in the skin applications transferring an electrical signal to the body via skin. Suitable applications are for example transcutaneous electrical nerve stimulation (TENS), electrical muscle stimulation (EMS), functional electrical stimulation (FES), wound care, electrosurgery, defibrillation, electrodermal activity and total body electrical conductivity.

More specifically, the sensing electrode according to the present invention is used in skin applications as a contact medium as part of electrodes measuring bio-signals from the skin. Suitable applications are for example electrocardiography (ECG), electroencephalography (EEG) and electromyography (EMG).

It is preferred that the relative vitality of the cells in the epidermis test for the dry stimulation electrode adhesive composition according to the present invention is more than 50% preferably more than 60% and more preferably more than 70%.

### Examples

### Example 1

### Screen printable acrylate-based adhesive comprising ionic liquid

250 g Loctite Duro-TAK 222A and 102.50 g diethylene glycol butyl ether was weighed in a round-bottom flask. A rotary evaporator was used for gentle removal of origin low-boiling point solvents in Loctite Duro-TAK 222A. Temperature was set to 40°C and air pressure was lowered stepwise from 1 bar to 40 mbar and remained 1 hour at 40 mbar. 91% of original solvent mixture was replaced by diethylene glycol butyl ether.

15 g of solvent-replaced Loctite Duro-TAK 222A was mixed with 0.224 g Basionics ST 80, 0.598 g 1-ethyl-3-methylimidazoliumtriflouromethansulfonate and 4.2 g Diethylene glycol butyl ether in a speed mixer for 2 minutes at 2000 rpm.

The formulation was manually screen-printed using screen mesh "SD 224/100" on top of Ag/AgCl film made of Loctite EDAG PE 409 (thickness: 20 µm, coated on PET Autostat CUS5). The printed layer was dried in conventional oven at 120° for 15 minutes, leading to an 25-30 µm thick conductive adhesive layer.

The ionically conductive adhesive layer on Ag/AgCl film was cut into two pieces, which were adhered together such that the connected area is 4 cm². An impedance of 1000 Ohm at 10 Hz was measured with a potentiostat from Metrohm Autolab.

### Example 2

### Screen printable acrylate-based adhesive comprising ionic liquid

250 g Loctite Duro-TAK 222A and 102.50 g Diethylene glycol butyl ether was weighed in a round-bottom flask. A rotary evaporator was used for gentle removal of origin low-boiling point solvents in Loctite Duro-TAK 222A. Temperature was set to 40°C and air pressure was lowered stepwise from 1 bar to 40 mbar and remained 1 hour at 40 mbar. 91% of original solvent mixture was replaced by diethylene glycol butyl ether.

15 g of solvent-replaced Loctite Duro-TAK 222A was mixed with 0.224 g Basionics ST 80, 0.598 g 1-ethyl-3-methylimidazoliumtriflouromethansulfonate, 0.56 g talcum (Mistron Monomix G) and 5.8 g diethylene glycol butyl ether in a speed mixer for 2 minutes at 2000 rpm.

The formulation was screen printed using screen mesh "SD 224/100" on top of Ag/AgCl film made of Loctite EDAG PE 409 (thickness: 20 µm, coated on PET Autostat CUS5). Printed layer was dried in conventional oven at 120° for 15 minutes, leading to an 25-30 µm thick conductive adhesive layer.

The ionically conductive adhesive layer on Ag/AgCl film was cut into two pieces, which were adhered together such that the connected area is 4 cm². An impedance of 1000 Ohm at 10 Hz was measured with a potentiostat from Metrohm Autolab.

### Example 3

### Screen printable acrylate-based adhesive comprising carbon black

132.88 g Loctite Duro-TAK 235A and 64.21 g diethylene glycol butyl ether was weighed in a round-bottom flask. A rotary evaporator was used for gentle removal of origin low-boiling point solvents in Loctite Duro-TAK 235A. Temperature was set to 40°C and air pressure has been lowered stepwise from 1 bar to 40 mbar and remained 1 hour at 40 mbar. 76% of original solvent mixture was replaced by diethylene glycol butyl ether.

50 g of solvent-replaced Loctite Duro-TAK 235A was mixed with 3.98 g Vulcan Xcmax22 (Cabot), and 9.56 g diethylene glycol butyl ether in a speed mixer for 2 minutes at 2000 rpm. The carbon black dispersion was improved with a triple roll mill (EXAKT, E80 Plus) at the lowest gap of 5 µm.

The formulation was screen printed using screen mesh "SD 224/100" on top of carbon film made of Loctite EDAG PF 407 (thickness: 15 µm, coated on PET Autostat CUS5). Printed layer was dried in conventional oven at 120° for 15 minutes, leading to an 35-40 µm thick conductive adhesive layer.

The electrically conductive adhesive layer on carbon film was cut into two pieces, which were adhered together such that the connected area is 4 cm². An impedance of 300 Ohm at 10 Hz was measured with a potentiostat from Metrohm Autolab.

### Example 4

### Screen printable acrylate-based adhesive comprising carbon black

76.2 g Loctite DURO-TAK UV 26105 and 76.2 g diethylene glycol butyl ether were weighed in a cylindrical aluminum cup and heated in an oven to 80°C, followed by mechanical stirring at 60°C (200 rpm) until completely dissolved.

20 g of dissolved Loctite UV 26105 was mixed with 1.59 g Vulcan Xcmax22 (Cabot), and 2.2 g diethylene glycol butyl ether in a speed mixer for 2 minutes at 2000 rpm. The carbon black dispersion was improved with a triple roll mill (EXAKT, E80 Plus) at the lowest gap of 5 µm.

The formulation was screen printed using screen mesh "SD 224/100" on top of carbon film made of Loctite EDAG PF 407 (thickness: 15 µm, coated on PET Autostat CUS5). Printed layer was dried in conventional oven at 120° for 15 minutes, leading to an 35-40 µm thick conductive adhesive layer.

The electrically conductive adhesive layer on carbon film was cut into two pieces, which were adhered together such that the connected area is 4 cm². An impedance of 1350 Ohm at 10 Hz was measured with a potentiostat from Metrohm Autolab.

### Example 5

### Polyurethane-based adhesive comprising ionic liquid

10 g Baymedix AR602 (Covestro AG), 0.987 g Baymedix AP501 (Covestro AG), 0.017g Borchi-Kat 0761, 0.146 g BYK 378 and 0.578 g 1-ethyl-3-methylimidazoliumtrifluoromethansulfonate was mixed in a speed mixer for 2 minutes at 2000 rpm.

Final formulation was coated on siliconized PET and cured in oven at 120°C for 20 minutes, leading to a 30 µm thick conductive adhesive film.

The ionically conductive adhesive was transferred on top of Ag/AgCl film made of Loctite EDAG PE 409 (thickness: 20 µm, coated on PET Autostat CUS5)and cut into two pieces, which were adhered together such that the connected area is 4 cm². An impedance of 1500 Ohm at 10 Hz was measured with a potentiostat from Metrohm Autolab.

### Example 6

### Thermoplastic polyurethane comprising carbon black

A thermoplastic PU PSA was prepared from 95.93 g Voranol CP 4755, 95.93 g PPG 400, 44.97 g Pharma Castor oil, 62.96 g 4,4' MDI, 0.12 g Byk A535, 0.09 g Fomrez UL 28 and 200 g ethyl acetate.

200 g 2-(2-butoxyethoxy) ethyl acetate were added to the solvent-based PSA in a round-bottom flask. A rotary evaporator was used for gentle removal of origin low-boiling point solvents in the solvent-based TPU. Temperature was set to 40°C and air pressure was lowered stepwise from 1 bar to 40 mbar and remained 1 hour at 40 mbar. >95% of original solvent mixture was replaced by diethylene glycol butyl ether.

10 g of solvent replaced TPU PSA was mixed with 1.06 g Vulcan XCmax22 (Cabot) in a speed mixer for 2 minutes at 2000 rpm. 4.6 g 2-(2-butoxyethoxy) ethyl acetate was added to lower to dilute the formulation to a solid content of 45wt.%. The dispersion of the carbon black was improved using a triple roll mill (EXAKT, E80 Plus) at the lowest gap of 0 µm.

For electrode preparation, the electrically conductive adhesive was transferred on top of a carbon film made of Loctite EDAG PF 407 (thickness: 20 µm, coated on PET Autostat CUS5).

### Example 7

### Thermoplastic polyurethane comprising ionic liquid

A thermoplastic PU PSA was prepared from 95.93 g Voranol CP 4755, 95.93 g PPG 400, 44.97 g Pharma Castor oil, 62.96 g 4,4' MDI, 0.12 g Byk A535, 0.09 g Fomrez UL 28 and 200 g ethyl acetate.

200 g 2-(2-butoxyethoxy) ethyl acetate were added to the solvent-based PSA in a round-bottom flask. A rotary evaporator was used for gentle removal of origin low-boiling point solvents in the solvent-based TPU. Temperature was set to 40°C and air pressure has been lowered stepwise from 1 bar to 40 mbar and remained 1 hour at 40 mbar. <95% of original solvent mixture was replaced by diethylene glycol butyl ether.

5 g of solvent-replaced TPU PSA (60wt.-% solid content) was mixed with 0.084 g Basionics ST 80, 0.251 g 1-ethyl-3-methylimidazoliumtriflouromethansulfonate in a speed mixer for 2 minutes at 2000 rpm.

For electrode preparation, the ionically conductive adhesive was transferred on top of Ag/AgCl film made of Loctite EDAG PE 409 (thickness: 20 µm, coated on PET Autostat CUS5).

## Claims

1. A stimulation or a sensing electrode comprising an ionically and/or electrically conductive pressure sensitive adhesive composition comprising
a) an ionic liquid and/or carbon black; and
b) a resin selected from the group consisting of a (meth)acrylate resin, a polyurethane resin, a silicone resin and mixtures thereof.

2. A stimulation or a sensing electrode according to claim 1, wherein the ionic liquid is selected from the group consisting of imidazolium acetates, imidazolium sulfonates, imidazolium chlorides, imidazolium sulphates, imidazolium phosphates, imidazolium thiocyanates, imidazolium dicyanamides, imidazolium benzoates, imidazolium triflates, choline triflates, choline saccharinate, choline sulfamates, pyridinium acetates, pyridinium sulfonates, pyridinium chlorides, pyridinium sulphates, pyridinium phosphates, pyridinium thiocyanates, pyridinium dicyanamides, pyridinium benzoates, pyridinium triflates, pyrrolidinium acetates, pyrrolidinium sulfonates, pyrrolidinium chlorides, pyrrolidinium sulphates, pyrrolidinium phosphates, pyrrolidinium thiocyanates, pyrrolidinium dicyanamides, pyrrolidinium benzoates, pyrrolidinium triflates, phosphonium acetates, phosphonium sulfonates, phosphonium chlorides, phosphonium sulphates, phosphonium phosphates, phosphonium thiocyanates, phosphonium dicyanamides, phosphonium benzoates, phosphonium triflates, sulfonium acetates, sulfonium sulfonates, sulfonium chlorides, sulfonium sulphates, sulfonium phosphates, sulfonium thiocyanates, sulfonium dicyanamides, sulfonium benzoates, sulfonium triflates, ammonium acetates, ammonium sulfonates, ammonium chlorides, ammonium sulphates, ammonium phosphates, ammonium thiocyanates, ammonium dicyanamides, ammonium benzoates, ammonium triflates and mixtures thereof.

3. A stimulation or a sensing electrode according to claim 1 or claim 2, wherein said ionic liquid is selected from the group consisting of 1-ethyl-3-methylimidazolium acetate, 1-ethyl-3-methylimidazolium methanesulfonate, 1-ethyl-3-methylimidazolium trifluoromethanesulfonate, 1-ethyl-3-methylimidazolium chloride, 1-ethyl-3-methylimidazolium ethyl sulphate, 1-ethyl-3-methylimidazolium diethylphosphate, 1-ethyl-3-methylimidazolium thiocyanate, 1-ethyl-3-methylimidazolium dicyanamide, 1-ethyl-3-methylimidazolium benzoate, choline trifluoromethanesulfonate, choline saccharinate, choline acesulfamate, choline *N*-cyclohexylsulfamate, tris(2-hydroxyethyl)methylammonium methyl sulphate, 1-ethyl-3-methylimidazolium tetrafluoroborate, 1-allyl-3-ethylimidazolium bis(trifluoromethylsulfonyl)imide, choline acetate, choline trifluoromethanesulfonate and mixtures thereof, and more preferably selected from the group consisting of 1-ethyl-3-methylimidazolium benzoate, 1-ethyl-3-methylimidazolium tetrafluoroborate, 1-ethyl-3-methylimidazolium methanesulfonate, 1-ethyl-3-methylimidazolium chloride, 1-ethyl-3-methylimidazolium trifluoromethanesulfonate, choline trifluoromethanesulfonate, 1-ethyl-3-methylimidazolium acetate, choline acetate, 1-ethyl-3-methylimidazolium diethylphosphate, 1-allyl-3-methylimidazolium bis(trifluoromethylsulfonyl)imide, 1-ethyl-3-methylimidazolium ethyl sulphate, 1-ethyl-3-methylimidazolium thiocyanate, 1-ethyl-3-methylimidazolium dicyanamide, choline saccharinate, choline acesulfamate, and mixture thereof.

4. A stimulation or a sensing electrode according to any one of claims 1 to 3, wherein said ionic liquid is present from 0.1 to 35% by weight of the total weight of the composition, preferably from 2 to 25%, and more preferably from 5 to 15%.

5. A stimulation or a sensing electrode according to any one of claims 1 to 4, wherein said carbon black has an oil absorption number from 70 ml/100g to 600 ml/100g, more preferably from 100 ml/100g to 500 ml/100g and more preferably from 150 ml/100g to 450 ml/100g, wherein said oil absorption number is measured according to ASTM D2414.

6. A stimulation or a sensing electrode according to any one of claims 1 to 5 wherein said carbon black has a iodine number preferably from 30 mg/g to 1700 mg/g, more preferably from 100 mg/g to 1500 mg/g and more preferably from 200 mg/g to 1400 mg/g, wherein said iodine number is measured according to ASTM D1510.

7. A stimulation or a sensing electrode according to any one of claims 1 to 6, wherein said carbon black is present from 0.01 to 30% by weight of the total weight of the composition, preferably from 1 to 25%, and more preferably from 5 to 20%.

8. A stimulation or a sensing electrode according to any one of claims 1 to 7 wherein said resin is a acrylate resin is formed from the monomers selected from the group consisting of methyl (meth)acrylate, ethyl (meth)acrylate, n-propyl (meth)acrylate, iso-propyl (meth)acrylate, n-butyl (meth)acrylate, iso-butyl (meth)acrylate, amyl (meth)acrylate, hexyl (meth)acrylate, ethylhexyl (meth)acrylate, octyl (meth)acrylate, isooctyl (meth)acrylate, decyl (meth)acrylate, dodecyl (meth)acrylate, hydroxyethyl (meth)acrylate, hydroxypropyl (meth)acrylate, hydroxybutyl (meth)acrylate, (meth)acrylic acid, benzyl (meth)acrylate, cyclohexyl (meth)acrylate, glycidyl (meth)acrylate, (meth)acrylamide, vinyl acetate, N-vinyl pyrrolidone, N-vinyl caprolactame, N-tertiary octyl acrylamide, dimethyl acrylamide, diacetone acrylamide, N-tertiary butyl acrylamide, N-isopropyl acrylamide, N-vinyl acetamide, N-vinyl formamide, acrylonitrile, vinyl ether and mixtures thereof, preferably selected from the group consisting of methyl (meth)acrylate, butyl (meth)acrylate, ethylhexyl acrylate, hydroxyethyl (meth)acrylate, hydroxypropyl (meth)acrylate, vinyl acetate, (meth)acrylic acid mixtures thereof.

9. A stimulation or a sensing electrode according to any one of claims 1 to 8 wherein said resin is a polyurethane resin formed from an isocyanate and polyol prepolymer.

10. A stimulation or a sensing electrode according to any one of claims 1 to 9 wherein said resin is a silicone resin or a silicone acrylate hybrid resin.

11. A stimulation or a sensing electrode according to any one of claims 1 to 10, wherein said resin is present from 30 to 98% by weight of the total weight of the composition, preferably from 50 to 95% and more preferably from 75 to 92%.

12. A stimulation or a sensing electrode according to any one of claims 1 to 11, wherein said adhesive has impedance value below 1,000,000 Ohm at 10 Hz, preferably below 100,000 Ohm at 10 Hz, and more preferably below 40,000 Ohm at 10 Hz, wherein said impedance is measured by connecting two electrodes coated each with 25 µm of an ionically and/or electrically conductive pressure sensitive adhesive having a contact area of 0.25 cm².

13. A stimulation or a sensing electrode according to any one of claims 1 to 12, wherein said electrode further comprises
a conductive layer, which is in contact with said ionically and/or electrically conductive pressure sensitive adhesive layer;
optionally a substrate, which is in contact with the conductive layer;
optionally a release liner, which is in contact with the conductive pressure sensitive adhesive layer; and
optionally a contact between said electrode and stimulation device.

14. Use of a stimulation or a sensing electrode according to any one of claims 1 to 13 in skin applications transferring an electrical signal to the body via the skin or in skin applications as a contact medium as part of electrodes measuring bio-signals from the skin.
